# EUROPEAN PATENT APPLICATION

(11) **EP 3 524 591 A1**
(43) Date of publication of application: **14.08.2019**
(21) Application number: 18156561.5
(22) Date of filing: 13.02.2018
(51) Int. Cl.: C07C 263/10, C07C 265/14

(54) **PROCESS FOR THE PREPARATION OF ISOCYANATES**

(71) Applicant: Covestro Deutschland AG, 51373 Leverkusen (DE); Covestro LLC, Pittsburgh, PA 15205-9741 (US)
(72) Inventor: The designation of the inventor has not yet been filed
(74) Representative: Levpat

(57) **Abstract**

The invention relates to a process for the preparation of aliphatic, cycloaliphatic or araliphatic isocyanates by reacting at least one primary organic amine with a stoichiometric excess of phosgene in the gas phase, comprising the steps a) reaction of the primary organic amine with an excess of phosgene in the gas phase and quenching the process product with a liquid comprising an inert aromatic solvent to obtain a liquid stream containing the isocyanate and a gas stream containing HCl and phosgene, b) separation of the gas stream containing HCl and phosgene obtained in step a) into a gas stream containing HCl and a liquid stream containing phosgene, c) partial vaporization of the liquid stream containing phosgene obtained in step b) to produce a gas stream containing phosgene, d) the gas stream containing phosgene obtained in step c) is at least partially recycled into the reaction in step a), and wherein the gas stream containing phosgene obtained in step c) contains 0.5 wt% or less of the sum of benzene, chlorobenzene and dichlorobenzene. The invention additionally relates to an isocyanate composition.

## Description

The invention relates to a process for the preparation of aliphatic, cycloaliphatic or araliphatic isocyanates by reacting primary organic amines with a stoichiometric excess of phosgene in the gas phase, wherein the excess phosgene is then recovered and recycled into the reaction. The invention additionally relates to an isocyanate composition.

Isocyanates are prepared in large quantities and are used mainly as starting materials for the preparation of polyurethanes. They are usually prepared by reacting the appropriate amines with phosgene, the latter being used in stoichiometric excess. The reaction of the amines with the phosgene can take place either in the gas phase or in the liquid phase. In these syntheses, at least part of the excess phosgene is normally obtained together with the gaseous hydrogen chloride by-product liberated in the reaction, so it is indispensable, for an economic operation of an isocyanate synthesis, to separate the excess phosgene from the hydrogen chloride by-product and recycle it into the reaction.

The present invention relates in particular to a process for recovery of the excess phosgene obtained in the preparation of isocyanates from amines and phosgene in gas-phase phosgenation, and for recycling of the recovered phosgene into the gas-phase reactor.

Various processes for the preparation of isocyanates by reacting amines with phosgene in the gas phase are known from the state of the art.

WO 2007/014936 A2 discloses a process for the preparation of diisocyanates by reacting diamines with a stoichiometric excess of phosgene, wherein at least part of the excess phosgene is recycled into the reaction and wherein the phosgene stream entering the reactor prior to mixing with the amine contains less than 15 wt% of HCl. Said patent document teaches that this is supposed to improve the working life of the reactors by reducing precipitations of amine hydrochlorides. A disadvantage of such high contents of inert HCl gas in the phosgene gas is that this entails large apparatuses and hence high plant construction costs. Furthermore, the inert HCl gas in the phosgene gas increases the circulating streams, resulting in increased operating costs. Thus it is generally always desirable to minimize the inert gas burden on the processes. An embodiment is described in which firstly the excess phosgene and the hydrogen chloride formed are separated from the essentially gaseous reaction mixture, and then at least part of the excess phosgene is recycled into the reaction, hydrogen chloride being separated from this recycled phosgene in such a way that the phosgene stream prior to mixing with the amine stream contains less than 15 wt% of HCl. The document describes that the separation is preferably carried out by means of a combination of distillation and washing: a detergent is used to wash the phosgene out of the stream containing hydrogen chloride, and the phosgene and hydrogen chloride are preferably separated from this loaded washing medium by distillation. According to the description, the washing and distillation can be operated at pressures of 1 to 10 bar absolute. The document does not disclose any requirements with regard to the solvent content of the phosgene stream entering the phosgenation reactor.

According to the teaching of WO 2008/086922 A1, in a gas-phase phosgenation reaction, the phosgene prior to mixing with the amine must not contain more than 1000 ppm by weight of chlorine, since otherwise there would be a risk of material embrittlement due to the high temperatures. According to this teaching, a certain amount of chlorine always forms due to the decomposition of phosgene at high temperatures, so it is necessary to separate this chlorine off. For this purpose the patent document discloses a procedure in which firstly the gaseous mixture containing phosgene, HCl and chlorine is subjected to partial condensation (p. 18,1. 30) and washing (p. 19,1. 18), in each case at a pressure of 0.1 to 20 bar absolute. This produces a liquid phase containing phosgene, washing medium, HCl and chlorine, from which the low boilers - chlorine and HCl - are then removed by rectification at a pressure of 1 to 5 bar absolute. In a subsequent step the phosgene and washing medium are separated from each other by rectification at a pressure of 1 to 5 bar absolute (p. 21, 1. 2), giving a phosgene stream of the desired chlorine purity which can be re-used in the phosgenation. According to the general teaching of this document, aromatic solvents, including monochlorobenzene or dichlorobenzene can be introduced together with the amine or phosgene into the gas phase reactor.

WO 2009/037179 A1 discloses a process for the preparation of isocyanates in the gas phase, wherein the phosgene is in essentially gaseous form in all the process steps, so it is no longer necessary to supply energy to evaporate liquid phosgene. According to the teaching of the patent document, this is achieved by a process in which the gaseous phosgene obtained in the phosgene production is introduced into the gas-phase phosgenation especially without intermediate condensation. Said document further describes a process for the separation of phosgene from a gaseous mixture with HCl and recycling of the separated phosgene into the gas-phase phosgenation by means of a combination of washing and multistage distillation operated under a pressure of 1 to 10 bar absolute. The document does not disclose any requirements with regard to the solvent content of the phosgene stream entering the phosgenation reactor.

WO 2011/003532 A1 discloses a process for preparing isocyanates by reaction of primary amines with phosgene in a stoichiometric excess in the gas phase, in which the excess phosgene is subsequently recovered and recirculated to the reaction. The recovery of phosgene from the gas mixture containing phosgene and hydrogen chloride is carried out in two stages. In the first step (hydrogen chloride-phosgene separation), the gas mixture containing hydrogen chloride and phosgene which leaves the reactor is separated into a gaseous stream containing mainly hydrogen chloride and a liquid stream containing phosgene and the liquid stream previously obtained is converted in a second step (phosgene gas production) into a gaseous, phosgene-containing stream, wherein the pressure in the first process step is lower than the pressure in the second process step. The process is advantageous since it allows the recovery of phosgene from the liquid, phosgene-containing scrubbing medium solution in only one step (phosgene gas production).

It is disclosed that an inert material, e.g. solvent, can be introduced into the gas-phase reaction. In fact, for the phosgene recycle stream, possible solvent contents extending up to 20 wt%, preferably between 5 ppm by weight and 10 wt% are described.

The recently published document WO 2017/009311 of the same assignee as the beforementioned WO 2011/003532 A1 teaches that it is beneficial for the overall process to allow higher concentrations, namely 1.0 to 15 wt%, of solvent in the gaseous phosgene stream that is recycled to the reactor.

A series of procedures by means of which mixtures of hydrogen chloride and phosgene and also possibly solvent can be fractionated in order to allow recirculation of the phosgene into the reaction are thus known from the prior art. However, the prior art is only concerned with the economics of reusing phosgene and HCl on the one hand and the burden of inerts being recycled in the process which leads to higher operating costs or in one case embrittlement of the equipment on the other hand. Problems caused by side reactions of recycled aromatic solvent into the reactor are not described in the state of the art.

Surprisingly, it has now been found that small amounts of benzene, chlorobenzene or dichlorobenzene entering the reaction zone may cause increased formation of polychlorinated aromatics in the gas phase phosgenation of aliphatic, cycloaliphatic or araliphatic diamines to form the corresponding diisocyanates. In particular, if the above mentioned solvents are present as impurities in the recycle phosgene, then polychlorinated aromatics, including hexachlorobenzene are formed, the latter being an unwanted compound. This effect on the formation of polychlorinated aromatics is surprising since comparably large amounts of the same solvent are used in the quench zone of the gas phase phosgenation process without forming polychlorinated aromatics and thus, the solvents have been considered as inert by those skilled in the art. On the contrary, there are many publications suggesting the use of aromatic solvents to support evaporation of the amines or dilute the phosgene.

According to the present invention the term "inert aromatic solvent" stands for an optionally substituted aromatic hydrocarbon which is at 20 °C and ambient pressure in a liquid state and which is inert towards isocyanate groups under the reaction conditions in step a). In case the aromatic hydrocarbon is chlorine substituted not more than two chlorine atoms are attached to the aromatic ring system.

It was therefore object of the present invention to provide a process which allows the reaction of aliphatic, cycloaliphatic or araliphatic diamines with phosgene in the gas phase to form the corresponding diisocyanates to be carried out in such a way that formation of polychlorinated aromatic compounds can be reduced.

This object has been achieved by a process for the preparation of aliphatic, cycloaliphatic or araliphatic isocyanates by reacting at least one primary organic amine with a stoichiometric excess of phosgene in the gas phase, comprising the steps
a) reaction of the primary organic amine with an excess of phosgene in the gas phase and quenching the process product with a liquid comprising an inert aromatic solvent to obtain a liquid stream containing the isocyanate and a gas stream containing HCl and phosgene,
b) separation of the gas stream containing HCl and phosgene obtained in step a) into a gas stream containing HCl and a liquid stream containing phosgene,
c) partial vaporization of the liquid stream containing phosgene obtained in step b) to produce a gas stream containing phosgene,
d) the gas stream containing phosgene obtained in step c) is at least partially recycled into the reaction in step a), and
wherein the gas stream containing phosgene obtained in step c) contains 0.5 wt% or less of the sum of benzene, chlorobenzene and dichlorobenzene.

### Gas-phase phosgenation (step a))

The phosgenation of amines in the gas phase by reaction with phosgene in step a) is generally known from the state of the art (e.g. WO 2007 014 936, WO 2008 086 922, WO 2011 003 532).

Suitable primary organic amines are aliphatic, cycloaliphatic or araliphatic amines, preferably diamines. More preferably the primary organic amine is selected from the group consisting of 1,6-diaminohexane (HDA), 1,5-diaminopentane (PDA), 1-amino-3,3,5-trimethyl-5-aminomethylcyclohexane (IPDA), 4,4'-diaminodicyclohexylamine, p-xylylenediamine and m-xylylenediamine, or mixtures of these amines, most preferably selected from the group consisting of HDA, PDA and IPDA.

Before the process according to the invention is carried out, the starting amines are normally evaporated and heated to 200°C to 600°C, preferably to 200°C to 500°C and particularly preferably to 250°C to 450°C, and fed into the reaction chamber optionally diluted with an inert gas such as N₂, He or Ar, or with the vapours of an inert solvent excluding inert aromatic solvents.

The starting amines can be evaporated in any of the known evaporation apparatuses. Preference is afforded to evaporation systems in which a small operating hold-up is passed with a high circulation capacity through a falling-film evaporator, where, to minimize the thermal stress on the starting amines, the evaporation process - as mentioned above - can optionally be supported by introducing inert gas and/or vapours of an inert solvent excluding inert aromatic solvents. Alternatively, the evaporation can also take place in special evaporation apparatuses with very short residence times, as described e.g. in EP 1 754 698.

In the process according to the invention, it is advantageous to use the phosgene in excess relative to the amine groups to be reacted. The molar ratio of phosgene to amine groups is preferably 1.1:1 to 20:1, particularly preferably 1.2:1 to 5:1 and most preferably 1.3:1 to 3.5:1. The phosgene is also heated to temperatures of 200°C to 600°C and fed into the reaction chamber optionally diluted with an inert gas such as N₂, He or Ar, or with the vapours of an inert solvent excluding inert aromatic solvents. The introduction of phosgene into the reactor can be done as a single stream comprising phosgene or by feeding a plurality of streams comprising phosgene. In each case the total feed stream to the reactor contains a fresh phosgene and recycled phosgene.

The process according to the invention is carried out in such a way that the separately heated reactants are introduced into at least one reaction chamber via at least one mixing device, mixed, and reacted, preferably adiabatically, while observing appropriate reaction times. The isocyanate is then condensed by cooling the gas stream down to a temperature above the decomposition point of the corresponding carbamic acid chloride, e.g. the acid chloride of hexamethylene diamine in the case of HDA.

The residence time required to react the amine groups with the phosgene to form isocyanate is between 0.01 and 15 seconds, preferably between 0.02 and 2 seconds, depending on the type of amine used, the starting temperature, the adiabatic temperature rise in the reaction chamber, the molar ratio of amine used to phosgene, any dilution of the reactants with inert gases, and the chosen reaction pressure.

After the phosgenation reaction has taken place in the reaction chamber, the gaseous reaction mixture, which preferably comprises at least one isocyanate, phosgene and hydrogen chloride, is freed of the isocyanate formed. This can be done e.g. by a procedure in which the mixture continuously leaving the reaction chamber, which preferably comprises at least one isocyanate, phosgene and hydrogen chloride, is condensed in an inert solvent after it has left the reaction chamber, in the manner already recommended for other gas-phase phosgenations (EP-A-0 749 958).

Preferably, however, the condensation is carried out as follows: The reaction chamber used in the process according to the invention has at least one zone into which one or more suitable liquid streams ("quenching liquors") are sprayed in order to stop the reaction between the amines used and the phosgene to form the corresponding isocyanates. According to the present invention, this liquid stream ("quenching liquors") is another term for the liquid comprising an inert aromatic solvent. Besides the inert aromatic solvents the quenching liquors can comprise different organic solvents which do not react with the diisocyanate formed. The inert aromatic solvent is preferably selected from the group consisting of benzene, chlorobenzene and dichlorobenzene or mixtures thereof, preferably selected from the group consisting of chlorobenzene and dichlorobenzene or a mixtures thereof. A solution of the diisocyanate formed in one of these organic solvents may also be used as quenching liquors. In this case, the proportion of solvent is preferably 40 to 90 vol%. The temperature of the quenching liquor is preferably 100 to 170 °C.

In one preferred embodiment of the process according to the invention, the throughput capacity of the reactor used with the reaction conditions required according to the invention is > 0.1 t amine/h, preferably 1 - 10 t amine/h. Throughput capacity is to be understood here as meaning that said throughput capacity of amine per h can be converted in the reactor.

Independently of the chosen type of cooling, the temperature of the at least one cooling zone is preferably chosen on the one hand so that it is above the decomposition point of the carbamoyl chloride corresponding to the isocyanate, and on the other hand so that the isocyanate and optionally the solvent concomitantly used as diluent in the amine vapour stream and/or phosgene stream condense as far as possible or dissolve in the solvent as far as possible, while excess phosgene, hydrogen chloride and inert gas optionally used concomitantly as diluent pass through the condensation or quenching stage as far as possible uncondensed or undissolved.

Particularly suitable for obtaining the isocyanate selectively from the gaseous reaction mixture are solvents like chlorobenzene and/or dichlorobenzene kept at a temperature of 80 to 200°C, preferably at 80 to 180°C, or the isocyanate or mixtures of the isocyanate with chlorobenzene and/or dichlorobenzene kept in these temperature ranges. It is easy for those skilled in the art to predict, on the basis of the physical data for a given temperature, pressure and composition, what proportion by weight of isocyanate condenses in the quencher or passes through the quencher uncondensed. Likewise, it is easy to predict what proportion by weight of excess phosgene, hydrogen chloride and inert gas optionally used as diluent passes through the quencher uncondensed or dissolves in the quenching liquor.

The gaseous mixture leaving the condensation or quenching stage is preferably freed of residual isocyanate with a suitable washing liquor in a downstream gas scrubber.

Preferably, the isocyanates are then purified by distillative work-up of the solutions or mixtures from the condensation or quenching stage. Thus, the inventive process preferably comprises a further step e) work-up of the liquid stream containing isocyanate obtained in step a) to isolate the desired isocyanate.

This workup is generally a multi-step distillative workup, carried out by known methods. In a first step, phosgene is removed, followed by the removal of solvent and low boiling impurities. In all these steps, the isocyanate is usually obtained in a bottom stream from the column. In the last refining step, isocyanate is distilled as a top product and thereby separated from high boiling impurities. The bottom stream from this last distillation stage can be subjected to a further concentration step in which more monomeric isocyanate is recovered from the bottom stream.

The essential step a) and the optional but preferably included step e) lead to an isocyanate or an isocyanate composition, since minor quantities of other compounds in the isocyanate composition cannot be fully excluded. Preferably, the isocyanate or isocyanate composition contains 10 ppm or less, preferably 3 ppm or less, more preferably 1 ppm or less, even more preferably 0.5 ppm or less and most preferably 0.3 ppm or less of hexachlorobenzene (in this invention also abbreviated as "HCB"). More preferably, the inventive isocyanate composition comprises an aliphatic, cycloaliphatic or araliphatic isocyanate and an amount of greater than zero to 10 ppm, preferably greater than zero to 3 ppm, more preferably greater than zero to 1 ppm, even more preferably greater than zero to 0.5 ppm and most preferably greater than zero to 0.3 ppm of hexachlorobenzene. Since the inventive isocyanate can contain minor amounts of additional compounds, it is also to be understood as an "isocyanate composition". Similar to the isocyanate above, this isocyanate composition contains essentially, preferably 99 wt% or more, based on the total amount of the isocyanate composition, of the desired isocyanate or mixtures of the desired isocyanates, preferably of the desired isocyanate.

According to the present invention, data in ppm are to be understood as being by weight (ppm by weight) unless stated otherwise.

The gas stream containing at least HCl and phosgene obtained from step a) is then separated in step b) into a gas stream containing HCl and a liquid stream containing phosgene.

### HCl/phosgene separation (step b))

According to the invention, the gaseous mixture leaving step a), containing at least HCl and the unreacted excess phosgene from the reaction, is separated, in the HCl/phosgene separation in step b), into a gas stream containing essentially HCl and a liquid stream containing phosgene.

Together with the reaction coupling product, HCl, and the unreacted excess phosgene, the gaseous mixture coming from step a) and entering the separation in step b) can optionally also contain inert gases and/or solvents and/or reaction by-products and/or traces of the reaction product. Examples of inert gases which may be mentioned are nitrogen, helium, argon, excess CO from the phosgene production, and CO₂. Examples of reaction by-products which may be mentioned are the by-products of the phosgene production, such as carbon tetrachloride, chloroform, monochloromethane, CO₂ and methane.

The gaseous mixture entering the separation in step b) normally contains 1 to 60 wt% of HCl, preferably 5 - 50 wt% of HCl and particularly preferably 10 - 45 wt% of HCl, based on the weight of gaseous mixture. This gaseous mixture normally contains 5 - 90 wt% of phosgene, preferably 15 - 85 wt% of phosgene, particularly preferably 25 - 80 wt% of phosgene and very particularly preferably 30 - 70 wt% of phosgene, based on the weight of gaseous mixture. The solvent content of the gaseous mixture is normally 0.01 - 60 wt%, preferably 0.05 - 40 wt% and particularly preferably 0.1 - 10 wt%, based on the weight of gaseous mixture. The solvent can be in either vapour or liquid form. The gaseous mixture can also contain inert gases normally totalling 0 - 10 wt%, preferably 0.0001 - 8 wt% and particularly preferably 0.001 - 5 wt%, based on the weight of gaseous mixture. The gaseous mixture can normally contain 0 - 10 wt%, preferably 0.001 - 7.5 wt% and particularly preferably 0.05 - 5 wt% of reaction product, based on the weight of gaseous mixture.

All the compositions given in this document are based on the weight of the particular components relative to the weight of the particular total stream, unless defined otherwise in the corresponding passages.

The separation according to the invention of the gas stream leaving step a), containing HCl and the unreacted excess phosgene from the reaction, can have various embodiments. One suitable method is partial condensation followed by washing. Complete or partial condensation followed by stripping is also suitable. Another suitable embodiment of this process step is absorption in a solvent. In particular, the absorption is effected in a solvent that is also used for the quenching. It is particularly preferable to use the same solvent as that used in the quenching step. Preferably this is chlorobenzene and/or dichlorobenzene.

In one preferred embodiment, step b) is carried out by absorption. In one particularly preferred embodiment, the absorption takes place in a sequence of at least 2 absorption steps, optionally in combination with partial condensation stages, at least one absorption step being carried out isothermally and at least one adiabatically. Very particularly preferably, the first absorption step is carried out isothermally and the following one adiabatically. According to another preference, the gas leaving the last absorption stage is further purified by condensing out residual traces of phosgene and solvent by cooling with a heat exchanger. In one preferred embodiment, the isothermal absorption and following adiabatic absorption are carried out in one apparatus, it also being particularly preferable to use the same apparatus to cool the gas stream leaving the absorption stage. This has the advantage of reducing the number of flanges and contributing to an increase in safety when handling phosgene. It also has the advantage of saving energy, since energy losses in the connecting pipelines are minimized by the compact design in one apparatus.

In one very particularly preferred embodiment, the gaseous mixture leaving step a) is partially condensed before entering the absorption stage, to give a liquid stream and a gas stream. Preferably, this condensation is carried out in such a way that the liquid stream contains phosgene, optionally solvents and only small amounts of dissolved HCl, and so that the gas stream contains HCl and optionally phosgene and inert gases. The gas stream obtained in the partial condensation is fed into the absorption stage. The condensation stage preferably takes place at temperatures of -40 - 0°C, particularly preferably at temperatures of -20 - 0°C. The condensation preferably takes place in a shell-and-tube heat exchanger, very preferably in a vertical shell-and-tube heat exchanger. Particularly preferably, the streams flow through the apparatus from top to bottom. Solvents can optionally be added to improve the condensing action. The solvent temperature is preferably below 10°C, particularly preferably below 0°C. The solvent may or may not contain phosgene.

In another very particularly preferred embodiment, the vapours from the condensation stage are subsequently passed in countercurrent through the solvent used in the quenche, whereby the phosgene, optionally together with traces of HCl and/or inert gases and/or reaction by-products, is absorbed in the solvent. Preferably, the gas rises through the absorption stages from bottom to top and the solvent runs through the absorption stages under gravity from top to bottom. In one particularly preferred embodiment, the liquid stream obtained in the condensation stage is combined at the bottom of the apparatus with the liquid streams flowing out of the absorption stages.

In another preferred embodiment, solvent at a temperature of -40 - 0°C, preferably of -20 to -10°C, is used for the adiabatic absorption step. According to a further preference, this solvent contains less than 1000 ppm, preferably less than 500 ppm and particularly preferably less than 250 ppm of phosgene. In one particularly preferred embodiment, the solvent already loaded with phosgene from the adiabatic absorption step is used for the isothermal absorption. However, it is also conceivable to carry out the isothermal absorption step either additionally or exclusively with other phosgene-containing solvent streams, e.g. those obtained in the distillation stage of phosgenation plants. In one preferred embodiment, the adiabatic temperature rise is 0.1 - 20°C, especially 2 - 5°C.

The amount of solvent introduced in the absorption step is 0.1 - 5 times, preferably 0.15 - 3 times, the weight of gaseous mixture entering process step a). The choice of introduced amount, temperature and composition of the solvent used, optionally in combination with adjustment of the process parameters, e.g. pressure and temperature in the HCl/phosgene separation, makes it possible to influence the quality of the gas stream exiting the absorption stage in step b) and the composition of the liquid stream containing phosgene leaving step b).

The isothermal absorption step is preferably carried out in a shell-and-tube heat exchanger, especially a vertical one. The liberated heat of absorption in the washing liquor is thereby transferred directly to the surface of the heat exchanger as it is produced, and dissipated. Preferably, the apparatus is cooled on the jacket side and the cooling medium enters at a temperature of -40 to 0°C, particularly preferably of -25 to -10°C. The number of tubes can vary within wide limits and is restricted only by the technical ability to manufacture them. To enlarge the contact area, the tubes can optionally be completely or partially filled with a filling material. Various appropriate packings or filling body systems are known to those skilled in the art.

The adiabatic absorption step that preferably follows the isothermal absorption step is preferably carried out in a column, which can be equipped with plates, packings or filling bodies. The adiabatic absorption step has preferably 1 to 50 theoretical plates, particularly preferably 2 to 40 theoretical plates.

In one preferred embodiment, the overall pressure loss over the isothermal and adiabatic absorption stages is less than 250 mbar, preferably less than 200 mbar and particularly preferably less than 150 mbar. This means that the pressure of the gas entering the isothermal absorption stage is not more than 250 mbar higher, preferably not more than 200 mbar higher and particularly preferably not more than 150 mbar higher than the pressure of the gas exiting the adiabatic absorption stage.

The liquid streams flowing out of the absorption stage(s) and condensation stage(s) preferably now have only a very small loading of dissolved HCl and/or dissolved inert gases and can be passed without further purification on to the phosgene gas production which is step c) according to the invention. Preferably, the streams flowing out of the condensation stage and the absorption stage are combined and passed as a common stream on to the phosgene gas production in step c).

Apart from phosgene, the liquid stream containing phosgene leaving step b) can normally also contain solvent and/or dissolved HCl and/or dissolved inert gases, optionally together with dissolved reaction by-products. This stream contains 25 - 90 wt%, preferably 30 - 80 wt%, particularly preferably 35 - 75 wt% and very particularly preferably 35 - 70 wt% of phosgene, based on the weight of liquid stream containing phosgene. This stream can also contain 10 - 75 wt%, preferably 15 - 70 wt% and particularly preferably 25 - 65 wt% of solvent, based on the weight of liquid stream containing phosgene, as well as 0 - 7 wt%, preferably 0.1 - 3.5 wt% and particularly preferably at most 0.5 - 3 wt% of dissolved HCl, based on the weight of liquid stream containing phosgene. This liquid stream can also optionally contain dissolved inert gases in a total amount of at most 1 wt%, preferably of at most 0.5 wt% and particularly preferably between 0.001 and 0.1 wt%, based on the weight of liquid stream containing phosgene. The content of any reaction by-products present is normally 0 - 5 wt%, preferably 0.001 - 3 wt% and particularly preferably 0.05 - 2.5 wt%, based on the weight of liquid stream containing phosgene.

The liquid stream containing phosgene exiting this process step is normally at a temperature of -40 to 20°C, preferably of -25 to 15°C, particularly preferably of -20 to 10°C and very particularly preferably of -15 to 8°C. On exiting the process step, said stream is normally under a pressure of 1 to 4 bara, preferably of 1.01 to 3 bara and particularly preferably of 1.02 to 2 bara. Exit from the process step for the liquid stream containing phosgene is understood as meaning the liquid discharge port of the apparatus(es) belonging to this process stage, the pressure measured at this point being corrected for the hydrostatic pressure of the liquid column in the apparatus(es).

The low content according to the invention of dissolved HCl and/or dissolved inert gas in the liquid stream containing phosgene produced in step b) has an advantageous effect in energy terms on the phosgene gas production in step c) because the total amount of gas to be produced in step c) is smaller as a result, so it requires a lower energy expenditure in step c). Moreover, the low content according to the invention of dissolved HCl and/or dissolved inert gas in the liquid stream containing phosgene produced in step b) does not create an intolerable inert gas burden in the downstream apparatuses along the phosgene gas path.

### Phosgene gas production (step c))

The liquid stream can be transferred from step b) to step c) continuously or batchwise, preferably it is transferred continuously by means of a pump.

According to the invention, the phosgene gas production in step c) is carried out in such a way that the liquid stream containing phosgene obtained from step b) is separated in step c) into a gas stream and a liquid stream. This can preferably be achieved by distillation or partial evaporation.

In one preferred embodiment, the phosgene gas production in step c) takes place in a distillation column with 1 - 80 theoretical plates, preferably 2 - 45 theoretical plates. The column can contain a stripping section and/or an enriching section, preferably both. Preferably, the stripping section has 1 - 40 theoretical plates, particularly preferably 1 - 20 theoretical plates, and the enriching section has 1 - 40 theoretical plates, particularly preferably 1 - 20 theoretical plates. The distillation column can be equipped with plates, packings or filling bodies, plates or packings being preferred. Suitable plates or packings are known to those skilled in the art, examples which may be mentioned, without implying a limitation, being sheet metal or woven fabric packings with structure, or bubble-cap, sieve or valve plates.

The column is normally operated at a bottom temperature of 100 to 250 °C, preferably of 110 to 230 °C and particularly preferably of 120 - 220 °C.

The differential pressure in the distillation column is normally smaller than 400 mbar, preferably smaller than 300 mbar and especially smaller than 200 mbar. Differential pressure is to be understood here as meaning the pressure difference between the top and bottom of the column.

In one preferred embodiment, the column is provided with a top condenser, which is particularly preferably inserted in the column. The top condenser is normally operated at a cooling medium entry temperature of -40 to 20 °C, preferably at -30 to 10 °C and particularly preferably at -25 to 0 °C. In one particularly preferred embodiment, the differential pressure of the gas across the top condenser is smaller than 150 mbar, particularly preferably smaller than 100 mbar. All or part of the condensate produced by the top condenser can be recycled into the column and/or withdrawn; preferably, all of the condensate is recycled into the column. The pressure of the gas stream leaving the condenser is preferably between 1.1 and 3.0 bara and the gas stream has a temperature below 30 °C, preferably below 25 °C, more preferably below 20 °C and very particularly preferably below 18 °C. Particularly preferably the pressure of the gas stream leaving the condenser is between 1.2 and 2.0 bara and the gas stream has a temperature below 30 °C, preferably below 25 °C, more preferably below 20 °C and very particularly preferably below 18 °C. Such vapor outlet conditions ensure a good separation of benzene, chlorobenzene and dichlorobenzene from the gas stream and thus minimize the recycling of such compounds to the reactor.

The energy supply at the bottom of the column can be provided by any conceivable evaporator, examples being natural-circulation evaporators, rising-film evaporators and falling-film evaporators. Falling-film evaporators are particularly preferred.

In one preferred embodiment, the liquid stream obtained from step b) is fed into the middle of the column, preferably between the enriching and stripping sections of the column.

In one particularly preferred embodiment, the column additionally has a top feed, said feed preferably being positioned above the enriching section. In one particularly preferred form, this is a liquid feed position. In one very particularly preferred embodiment, liquid phosgene is introduced through this feed position. In a most preferred embodiment, the liquid phosgene that is introduced to this feed position is fresh phosgene.

In terms of the present patent application, fresh phosgene is understood as meaning phosgene which does not originate directly from the process according to the invention and that has a mass fraction of inert aromatic solvents 0.5 wt% or less, preferably 0.35 wt% or less of inert aromatic solvent, more preferably 0.15 wt% or less of inert aromatic solvent, most preferably 0.05 wt% or less of inert aromatic solvent. It is preferably phosgene which, after the phosgene synthesis, usually from chlorine and carbon monoxide, does not pass through a reaction stage with a phosgene conversion of more than 5% of the conversion of the phosgene prepared in the phosgene synthesis.

The liquid phosgene optionally fed into the top of the column is normally at a temperature of -30 to 10 °C, preferably of -20 to 0 °C. This stream normally contains essentially phosgene, i.e. the phosgene content is between 95 and 100 wt%; preferably, the phosgene content is between 98 and 100 wt%, based on the weight of this stream.

According to another preferred embodiment, step c) is carried out in a device comprising at least one distillation column having preferably an additional top feed, more preferably an additional top feed positioned above the enriching section and most preferably an additional liquid top feed positioned above the enriching section.

In this embodiment it is further preferred that a liquid, preferably liquid phosgene and more preferably liquid fresh phosgene is introduced through the additional top feed of the distillation column. Such a feed of liquid phosgene can further reduce the content of inert aromatic solvents in the phosgene gas stream exiting the phosgene gas generation step.

The combined mass fraction of benzene, chlorobenzene and dichlorobenzene in this stream is 0.5 wt% or less, preferably 0.35 wt% or less, more preferably 0.15 wt% or less and most preferably 0.05 wt% or less.

In another embodiment, the column can additionally have a feed position for a gas stream. This feed position is preferably located below or above the enriching section or else below the stripping section.

In another possible embodiment, the phosgene gas production in step c) is carried out in such a way that the liquid stream containing phosgene from step b) is separated by partial evaporation into a gas stream containing phosgene and optionally inert gases, and a liquid stream. For this purpose the liquid stream obtained from step b) is fed into an evaporator, which is heated by an external heating medium. The bottom temperature of the evaporator is in the range from 30 to 250°C, preferably from 70 to 230°C and particularly preferably in the range 100 - 220°C.

In addition to the liquid stream from step b), another liquid phosgene stream can also be introduced into the evaporator. This other liquid phosgene stream is normally at a temperature of -30 to 10°C, preferably of -20 to 0°C. The stream normally contains essentially phosgene, i.e. the phosgene content is between 95 and 100 wt%; preferably, the phosgene content is between 98 and 100 wt%, based on the weight of this stream. The combined mass fraction benzene, chlorobenzene and dichlorobenzene in this stream is preferably 0.5 wt% or less, particularly preferably 0.2 wt% or less and most preferably 0.1 wt% or less.

The liquid is partially evaporated in the evaporator, i.e. there is a discontinuous or, preferably, continuous discharge of liquid from the evaporator.

It is further possible to support the phosgene gas production in the various embodiments, e.g. by adding inert gases such as nitrogen.

The gas stream obtained in the phosgene gas production in step c) contains essentially phosgene. Apart from phosgene, this stream can also contain inert gases and/or solvents and/or reaction by-products and/or HCl. According to the invention, this stream contains less than 0.5 wt%, preferably less than 0.35 wt% and particularly preferably less than 0.15 wt%, most preferably less than 0.05 wt% of benzene, chlorobenzene and dichlorobenzene (limits are given for the sum of these compounds). To optimize the energy input, it is reasonable to allow this gas stream to have a certain solvent content, preferably 0.002 wt% or more and preferably 0.005 wt% or more of the sum of benzene, chlorobenzene and dichlorobenzene.

The sum of benzene, chlorobenzene and dichlorobenzene in wt% can be determined by standard gas chromatographic analysis using an internal standard after neutralizing the sample and extraction with a suitable organic solvent. Preferably, a gas sample is collected in a previously evacuated stainless steel bomb and then bubbled through aqueous NaOH to decompose phosgene and neutralize any HCl present in the gas mixture. The bomb is rinsed twice with a known amount of o-dichlorobenzene which is then added to the aqueous mixture. The mixture is neutralized with IN HCl to pH 7. The organic contents (including benzene chlorobenzene and dichlorobenzene) are extracted into o-dichlorobenzene by thoroughly mixing the aqueous solution with a known amount of o-dichlorobenzene and separating the liquid layers using a separatory funnel. The organic layer is tested by GC (DB-17 column with dimensions 30 m x 0.25 mm x 0.25 µm; carrier gas helium; constant flow 1.1 mL/min; split ratio 100:1 with a split flow of 110 mL/min; inlet temperature 250 °C; oven temperature held at 45 °C for 10 min, then increased at 10 °C/min to 135 °C with a hold time of 13 min followed by further increase at 20 °C/min to 220 °C with a hold time of 12 min; FID detector with 400 mL/min oxidizer, 40 mL/min of hydrogen with makeup combo flow of 30 mL/min, makeup gas helium) against internal standards to determine the concentration of benzene and chlorobenzene and thereby the amount of each of these components that was present in the gas sample. From these amounts and the weight of the gas sample, the corresponding concentrations of benzene and chlorobenzene in wt% can easily be calculated. For the determination of the dichlorobenzene (all isomers) content, the procedure is repeated with the difference that in this case chlorobenzene is used to rinse the stainless steel bomb and extract the organics from the aqueous solution. Adding up the so derived concentrations of benzene, chlorobenzene and dichlorobenzene isomers gives the sum of benzene, chlorobenzene and dichlorobenzene as it is referred to in this document.

This stream preferably contains a total of at most 1 wt%, particularly preferably at most 0.5 wt% and most preferably 0.1 wt% of inert gases, based on the weight of the gas stream.

Preferably it can also contain at most 20 wt%, more preferably at most 10 wt% and particularly preferably at most 5 wt% of HCl, based on the weight of the gas stream. The content of any reaction by-products present is normally up to 5 wt%, preferably up to 4 wt% and particularly preferably up to 2.5 wt%, based on the weight of the gas stream.

The pressure of the gas stream leaving the condenser is preferably between 1.1 and 3.0 bara and the gas stream has a temperature below 30 °C, preferably below 25 °C, more preferably below 20 °C and very particularly preferably below 18 °C. Particularly preferably the pressure of the gas stream leaving the condenser is between 1.2 and 2.0 bara and the gas stream has a temperature below 30 °C, preferably below 25 °C, more preferably below 20 °C and very particularly preferably below 18 °C. Such vapor outlet conditions ensure a good separation of inert aromatic solvents from the gas stream and thus minimize the recycling of such compounds to the reactor.

The gas stream containing phosgene obtained in the phosgene gas production in the process according to the invention is normally at a temperature of -10 - 30°C, preferably of 0 - 25°C, particularly preferably of 5 - 20°C and most preferably of 7 to 18 °C on exiting this process step. The gas stream can then be heated up before being recycled into step a). The pressure of the gas stream obtained is normally 1.05 to 6.0 bara, preferably 1.1 to 3.0 bara and particularly preferably 1.2 to 2.0 bara on exiting this process step. Exit from the process stage is understood as meaning the gas discharge port of the apparatus(es) in which step c) is carried out.

The liquid stream obtained in the phosgene gas production in step c) consists essentially of solvent. In addition to the latter, this stream can also contain reaction by-products. It can further contain certain amounts of phosgene. This liquid stream normally contains 80 - 100 wt%, preferably 85 - 99.95 wt%, particularly preferably 90 - 99.9 wt% and very particularly preferably 95 - 99.8 wt% of solvent, based on the weight of the liquid stream.

This stream can also contain up to 20 wt%, preferably up to 15 wt%, particularly preferably up to 10 wt% and very particularly preferably up to 5 wt% of dissolved phosgene, based on the weight of the liquid stream. To optimize the energy input, it is reasonable to allow this liquid stream to have a certain phosgene content, which is normally at least 1 ppm by weight, preferably at least 3 ppm by weight and particularly preferably at least 8 ppm by weight, based on the weight of the liquid stream. This stream is normally loaded with a total of at most 0.5 wt%, preferably at most 0.1 wt% and particularly preferably 0.05 wt% of dissolved inert gases, based on the weight of the liquid stream. It can also contain at most 1 wt%, preferably at most 0.1 wt% and particularly preferably at most 0.05 wt% of HCl, based on the weight of the liquid stream. The content of any reaction by-products present is normally up to 5 wt%, preferably up to 4 wt% and particularly preferably up to 2.5 wt%, based on the weight of the liquid stream.

Preferably, the liquid stream obtained from step b) is passed indirectly on to the phosgene gas production in step c). In terms of this patent, indirectly means that one or more unit operations, for example heat transfers, pressure changes, changes in composition are performed on the stream. Particularly preferably, the temperature of the liquid stream is changed, preferably raised. The increase in temperature of the stream between the exit from step b) and the entry into step c) normally being between 0.5 and 220°C, preferably between 1 and 200°C and particularly preferably between 5 and 175°C.

Particularly preferably, the temperature is raised by exchange with at least one other liquid material stream in the plant. This exchange preferably takes place in a heat exchanger such as a shell-and-tube heat exchanger or a plate-type heat exchanger, preferably a shell-and-tube heat exchanger.

All or part of the liquid stream obtained in step c) can optionally be used as solvent in the HCl/phosgene separation in step b). This is particularly advantageous for removing low-boiling reaction products, together with the gas stream obtained in step b), from the process.

The process according to the invention makes it possible to achieve a high phosgene recovery yield. Phosgene recovery yield is understood as meaning the proportion of phosgene which, via step b) according to the invention, is separated from the gaseous mixture leaving the reactor, containing at least HCl and the unreacted excess phosgene from the reaction, and which, via the gas stream obtained in step c), is recycled into the reaction according to step a).

The phosgene recovery yield is calculated by forming the quotient in percent of the amount of phosgene in the gas stream entering process step b) and the amount of phosgene in the gas stream exiting process step c), and subtracting any fresh phosgene that has been fed in.

In general, the phosgene recovery yield is more than 90%, especially more than 93%, preferably more than 95% and particularly preferably more than 98%.

### Phosgene recycling (step d))

All or part of the gas stream obtained from step c) in the process according to the invention is recycled into the reaction according to step a). Preferably, the entire stream is recycled into the reaction according to step a). In particular, it is not necessary to recycle part of the gas stream obtained in step c) into the HCl/phosgene separation (step b)).

The fresh phosgene required for the phosgenation, i.e. the phosgene normally produced by reacting chlorine with carbon monoxide, can be introduced into the process according to the invention in different ways.

On the one hand it is possible to use the fresh phosgene in gaseous form. The gaseous fresh phosgene, optionally in combination with a gaseous stream obtained in the phosgene production in step c), can be passed, together or separately, to the reactors for the phosgenation reaction in step a).

A further possibility is to pass the fresh phosgene in gaseous form on to the phosgene gas production in step c). This is advantageous because the introduction of gas facilitates the evaporation of the liquid stream in this process step due to the stripping effect.

On the other hand it is possible first to liquefy the fresh phosgene, thereby purifying it by removing as much as possible of the inert gases and by-products of the phosgene preparation. In this case the resulting liquid phosgene can be passed on to the HCl/phosgene separation in step b). However, it is also possible to pass this liquid phosgene on to the phosgene gas production in step c). A further possibility is for the liquid phosgene produced in this way to be evaporated in a separate apparatus to give a gaseous phosgene stream, which can be introduced into the process according to the invention in accordance with the possibilities described in the previous paragraph.

Another subject of the present invention is an aliphatic, cycloaliphatic or araliphatic isocyanate, obtainable or obtained by the inventive process. These products are linked with the beneficial effect that especially the amount of polychlorinated aromatics is significantly reduced compared to the prior art products. Preferably, the isocyanate contains 10 ppm or less, preferably 3 ppm or less, more preferably 1 ppm or less, even more preferably 0.5 ppm or less and most preferably 0.3 ppm or less of hexachlorobenzene. Even more preferably, the isocyanate contains an amount of greater than zero to 10 ppm, preferably greater than zero to 3 ppm, more preferably greater than zero to 1 ppm, even more preferably greater than zero to 0.5 ppm and most preferably greater than zero to 0.3 ppm of hexachlorobenzene. Since the inventive isocyanate can contain minor amounts of additional compounds, it is also to be understood as an "isocyanate composition". Similar to the isocyanate above, this isocyanate composition contains essentially, preferably 99 wt% or more, based on the total amount of the isocyanate composition, of the desired isocyanate or mixtures of the desired isocyanates, preferably of the desired isocyanate.

The HCB content can be determined by a GC-MS method with reference to DIN 54232:2010-08. The isocyanate sample is then treated as described for the hackled textile sample and the GC is operated in constant flow mode with a split ratio of 25: 1. The method allows detection of HCB down to 100 ppb or even below 100 ppb.

Another subject of the present invention is an isocyanate composition containing an aliphatic, cycloaliphatic or araliphatic isocyanate and 10 ppm or less, preferably 3 ppm or less, more preferably 1 ppm or less, even more preferably 0.5 ppm or less and most preferably 0.3 ppm or less of hexachlorobenzene, determined by GC-MS according to DIN 54232:2010-08. Preferably, the inventive isocyanate composition comprises an aliphatic, cycloaliphatic or araliphatic isocyanate and an amount of greater than zero to 10 ppm, preferably greater than zero to 3 ppm, more preferably greater than zero to 1 ppm, even more preferably greater than zero to 0.5 ppm and most preferably greater than zero to 0.3 ppm of hexachlorobenzene, determined by GC-MS according to DIN 54232:2010-08. For determination of the hexachlorobenzene content, the isocyanate composition sample is treated as described in DIN 54232:2010-08 for the hackled textile sample and the GC is operated in constant flow mode with a split ratio of 25:1.

The inventive composition is to be understood as a composition comprising essentially, preferably 99 wt% or more, based on the total amount of the isocyanate composition, of the desired isocyanate or mixtures of the desired isocyanates and the given amounts of hexachlorobenzene. The isocyanate composition may contain other components but preferably they are present in an amount as low as possible. However, the effort necessary for complete removal should be balanced with their influence on the final product.

The isocyanate in the isocyanate composition is derived from the suitable and preferred amines disclosed above in step a) of the inventive process.

In a preferred embodiment the isocyanate is a diisocyanate. More preferably the isocyanate is selected from the group consisting of 1,6-diisocyanatohexane (HDI), 1,5-diisocyanatopentane (PDI), 1-isocyanato-3,3,5-trimethyl-5-isocyanatomethyl-cyclohexane (IPDI), 4,4'-diisocyanatodicyclohexylamine, p-xylylenediisocyanate and m-xylylenediisocyanate, or mixtures of these isocyanates, most preferably selected from the group consisting of HDI, PDI and IPDI.

In another preferred embodiment, the isocyanate is an aliphatic diisocyanate, more preferably selected from the group consisting of HDI and PDI.

The invention will be illustrated below with the aid of examples and comparative examples, but without being restricted thereto.

### Examples:

Data in ppm are to be understood as being by weight (ppm by weight). Data in mbara or bara denote the absolute pressure in mbar or bar, respectively.

The HCB content in refined isocyanate was determined by GC-MS method with reference to DIN 54232:2010-08. The isocyanate sample was treated as described for the hackled textile sample and the GC was set to constant flow mode with a split ratio of 25:1.

The liquid streams containing the isocyanate produced in step a) were worked up by distillation in the same manner for all three examples. In a first step, remaining phosgene was separated from the liquid crude product by distillation. The resulting product stream taken from the bottom of the column comprised isocyanate, chlorobenzene and other impurities formed during the reaction. In the next step, solvent was removed in a pre-evaporation stage followed by a solvent/low boiler distillation, resulting in a liquid bottom stream that contained mainly the isocyanate and some high boiling impurities. Finally this bottom stream was distilled in a last column to obtain the desired isocyanate as a head product. All process conditions like flow rates, reflux ratios, temperatures and pressures were identical within the precision of the measurements for all three examples. The changes in the HCB content of the product can therefore not be attributed to changes in the workup procedure of the liquid stream containing the isocyanate. Instead, it has to be a result of a lower formation rate for the HCB and consequently a lower content of HCB already in the crude reaction product.

Method for the determination of inert aromatic solvents in the phosgene recycle gas stream leaving step c):
A first gas sample is collected in a previously evacuated stainless steel bomb and then bubbled through aqueous NaOH to decompose phosgene and neutralize any HCl present in the gas mixture. The bomb is rinsed twice with a known amount of o-dichlorobenzene which is then added to the aqueous mixture. The mixture is neutralized with IN HCl to pH 7. The organic contents (including benzene chlorobenzene and dichlorobenzene) are extracted into o-dichlorobenzene by thoroughly mixing the aqueous solution with a known amount of o-dichlorobenzene and separating the liquid layers using a separatory funnel. The organic layer is tested by GC (DB-17 column with dimensions 30 m x 0.25 mm x 0.25 µm; carrier gas helium; constant flow 1.1 mL/min; split ratio 100:1 with a split flow of 110 mL/min; inlet temperature 250 °C; oven temperature held at 45 °C for 10 min, then increased at 10 °C/min to 135 °C with a hold time of 13 min followed by further increase at 20 °C/min to 220 °C with a hold time of 12 min; FID detector with 400 mL/min oxidizer, 40 mL/min of hydrogen with makeup combo flow of 30 mL/min, makeup gas helium) against internal standards to determine the concentration of benzene and chlorobenzene and thereby the amount of each of these components that was present in the gas sample. From these amounts and the weight of the gas sample, the corresponding concentrations of benzene and chlorobenzene in wt% can easily be calculated.
A second gas sample is treated in an analogous way with the exception that chlorobenzene is used instead of o-dichlorobenzene and the contents of dichlorobenzene isomers are determined.

Adding up the so derived concentrations of benzene, chlorobenzene and dichlorobenzene isomers gives the sum of benzene, chlorobenzene and dichlorobenzene.

### Example 1 (according to the invention):

In a tubular reactor with downstream isocyanate condensation stage (quench with liquid MCB/HDI mixture), gaseous hexamethylene diamine was mixed with a phosgene gas stream via a mixing nozzle and reaction took place in the gaseous state immediately after mixing. The reactor pressure was 1600 mbara and the adiabatic reaction temperature reaches >400 °C. After quenching, a liquid stream containing hexamethylene diisocyanate and a gas stream containing phosgene and HCl was obtained. Both streams also contained monochlorobenzene. The stream containing isocyanate was purified by distillation to remove high-boiling and low-boiling impurities, giving purified hexamethylene diisocyanate containing 7 ppm of hexachlorobenzene.

The gas stream containing HCl and phosgene was separated by the following procedure into an HCl gas stream and a liquid stream containing phosgene: First the stream was passed from bottom to top through an absorption column with cold condensed MCB stream flowing from top to bottom, thereby maximizing the absorption of phosgene from the gas stream. The residual gas exiting the top condenser was then passed through an isothermal absorption step followed by an adiabatic absorption step. Also, cold solvent at a temperature of -10 °C was fed into the top of the adiabatic absorption step and flows through the absorption steps in countercurrent with the gas. HCl gas was withdrawn from the top of the absorption column and a solution consisting of MCB and phosgene was obtained at the liquid level at the bottom of the absorption column under a pressure of approx. 1350 mbara and a temperature of -10 °C.

The resulting phosgene solution was pumped into the phosgene gas production, which was carried out in the form of a desorption column. The top of the column was equipped with an internal condenser that was operated with a chilled cooling medium. The inlet temperature of the cooling medium was -17 °C. The inflow of the phosgene solution was situated between the stripping and enriching sections of the column. A gas stream containing ca. 95 wt% of phosgene and 5 wt% of HCl was withdrawn from the top of the column under a pressure of 1700 mbara and with an outlet temperature of 28 °C. This stream was mixed in gaseous form with a phosgene gas stream (fresh phosgene) from the phosgene production and conveyed to the tubular reactor for reaction with the amine. A sample of the recycled phosgene gas stream was analysed for its contents of benzene (none detected), chlorobenzene (0.5 wt%) and dichlorobenzene (none detected) and a total of 0.5 wt% was detected for the sum of the three compounds.

### Example 2 (according to the invention):

The same setup as in example 1 was used. The chilled cooling medium flow in the desorption column top condenser has been increased so that the vapor outlet temperature dropped to 22 °C.

A sample of the recycled phosgene gas stream was analysed for its contents of benzene (none detected), chlorobenzene (0.24 wt%) and dichlorobenzene (none detected) and a total of 0.24 wt% was detected for the sum of the three compounds.
The distilled HDI stream obtained from the process contained only 300 ppb of hexachlorobenzene.

### Example 3 (comparative example):

The same setup as in example 1 was used. The chilled cooling medium flow in the desorption column top condenser has been decreased so that the vapor outlet temperature increased to 32 °C.

A sample of the recycled phosgene gas stream was analysed for its contents of benzene (none detected), chlorobenzene (0.65 wt%) and dichlorobenzene (38 ppm by weight) and a total of 0.65 wt% was detected for the sum of the three compounds.
The distilled HDI stream obtained from the process contained 11 ppm of hexachlorobenzene.

## Claims

1. Process for the preparation of aliphatic, cycloaliphatic or araliphatic isocyanates by reacting at least one primary organic amine with a stoichiometric excess of phosgene in the gas phase, comprising the steps
a) reaction of the primary organic amine with an excess of phosgene in the gas phase and quenching the process product with a liquid comprising an inert aromatic solvent to obtain a liquid stream containing the isocyanate and a gas stream containing HCl and phosgene,
b) separation of the gas stream containing HCl and phosgene obtained in step a) into a gas stream containing HCl and a liquid stream containing phosgene,
c) partial vaporization of the liquid stream containing phosgene obtained in step b) to produce a gas stream containing phosgene,
d) the gas stream containing phosgene obtained in step c) is at least partially recycled into the reaction in step a), and
wherein the gas stream containing phosgene obtained in step c) contains 0.5 wt% or less of the sum of benzene, chlorobenzene and dichlorobenzene.

2. Process according to Claim 1, wherein the gas stream containing phosgene obtained in step c) contains 0.35 wt% or less, preferably 0.15 wt% or less and more preferably 0.05 wt% or less of the sum of benzene, chlorobenzene and dichlorobenzene.

3. Process according to Claim 1 or 2, wherein the gas stream containing phosgene obtained in step c) contains 0.002 wt% or more, preferably 0.005 wt% or more of the sum of benzene, chlorobenzene and dichlorobenzene.

4. Process according to any of the preceding claims, wherein the inert aromatic solvent is selected from the group consisting of benzene, chlorobenzene and dichlorobenzene or mixtures thereof, preferably selected from the group consisting of chlorobenzene and dichlorobenzene or mixtures thereof.

5. Process according to any of the preceding claims, wherein the process comprises a further step e) work-up of the liquid stream containing isocyanate obtained in step a) to isolate the desired isocyanate.

6. Process according to any of the preceding claims, wherein the primary organic amine is selected from the group consisting of 1,6-diaminohexane (HDA), 1,5-diaminopentane (PDA), 1-amino-3,3,5-trimethyl-5-aminomethylcyclohexane (IPDA), 4,4'-diaminodicyclohexylamine, p-xylylenediamine and m-xylylenediamine, or mixtures of these amines, preferably selected from the group consisting of HDA, PDA and IPDA.

7. Process according to any of the preceding claims, wherein the pressure of the gas stream containing phosgene obtained in step c) is between 1.1 and 3.0 bara and the gas stream has a temperature below 30 °C, preferably below 25 °C, more preferably below 20 °C and very particularly preferably below 18 °C.

8. Process according to any of the preceding claims, wherein step c) is carried out in a device comprising at least one distillation column having preferably an additional top feed, more preferably an additional top feed positioned above the enriching section and most preferably an additional liquid top feed positioned above the enriching section.

9. Process according to claim 8, wherein a liquid, preferably liquid phosgene and more preferably liquid fresh phosgene is introduced through the additional top feed of the distillation column.

10. Isocyanate composition containing an aliphatic, cycloaliphatic or araliphatic isocyanate and 10 ppm or less, preferably 3 ppm or less, more preferably 1 ppm or less, even more preferably 0.5 ppm or less and most preferably 0.3 ppm or less of hexachlorobenzene, determined by GC-MS according to DIN 54232:2010-08.

11. Isocyanate composition according to claim 10, wherein the isocyanate is a diisocyanate.

12. Isocyanate composition according to claim 11, wherein the isocyanate is an aliphatic diisocyanate.
